(19) Europäisches Patentamt
European Patent Office
Office européen des brevets

(11) EP 3 973 861 A1

(12) EUROPEAN PATENT APPLICATION
published in accordance with Art. 153(4) EPC

(43) Date of publication:
30.03.2022 Bulletin 2022/13

(51) International Patent Classification (IPC):
A61B 5/0476 (2006.01)   G06F 3/01 (2006.01)

(21) Application number: 20809757.6

(52) Cooperative Patent Classification (CPC):
A61B 5/369; G06F 3/01

(22) Date of filing: 22.05.2020

(86) International application number:
PCT/JP2020/020342

(87) International publication number:
WO 2020/235680 (26.11.2020 Gazette 2020/48)

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME
Designated Validation States:
KH MA MD TN

(30) Priority: 23.05.2019 JP 2019097202

(71) Applicant: Nitta, Tsuneo
Yokohamai-shi, Kanagawa, 247-0009 (JP)

(72) Inventor: Nitta, Tsuneo
Yokohamai-shi, Kanagawa, 247-0009 (JP)

(74) Representative: Peters, Sebastian Martinus
Octrooibureau Vriesendorp & Gaade B.V.
Koninginnegracht 19
2514 AB Den Haag (NL)

(54) SPEECH IMAGERY RECOGNITION DEVICE, WEARING FIXTURE, SPEECH IMAGERY RECOGNITION METHOD, AND PROGRAM

(57) Provided is a speech imagery recognition device which enables spoken language to be recognized by means of brain waves. The present invention provides a speech imagery recognition device 1 for recognizing spoken language from brain waves during speech imagery, and includes: a brain wave input unit 2 for converting brainwaves input from an electrode group 22 into discrete signal groups; an analysis processing unit 4 for subjecting the discrete signal groups of brain waves for each electrode, input from the electrode group 22, to analysis processing, and outputting spectrum time series; a language characteristic extracting unit 5 for outputting a phoneme characteristic vector time series on the basis of the spectrum time series; a word/sentence recognizing unit for recognizing the spoken language on the basis of the phoneme characteristic vector time series; and a post-processing/output unit 7 for outputting the spoken language recognized by the word/sentence recognizing unit 6.

FIG. 1

**Description**

[Technical Field]

The present invention relates to a speech imagery recognition device, a wearing fixture, a speech imagery recognition method, and a program.

[Background Art]

A speech input device that is in practical use receives speech sound waves from a microphone or bone conduction vibrations from a vibration pickup to recognize speech information from the signals obtained.

Recently, using a huge amount of voice and language data, probability information about the sequence of phonemes (acoustic model) and the sequence of words (language model) is stored and used on a network, which achieves high-speed and high-performance speech recognition. Meanwhile, due to annoyance to others caused by speaking and the leakage of information as well as the increase in the number of patients with amyotrophic lateral sclerosis (ALS) who have difficulty in speaking, language recognition is desired to be carried out based on speech imagery without speech production in the field of brain-computer interface (BCI).

Speech recognition of spoken words has lately been attempted through speech imagery signals by monitoring 64 to 128-channel electrocorticographic (ECoG) recordings from the cerebral cortex (see Non-Patent Document 1). However, it is not realistic to use such a method involving craniotomy for other than critically ill patients. Besides, although a technology that uses electrodes placed along the scalp to record an electroencephalogram (EEG) will make an invaluable contribution to society when put to practical use, attempts to find meaningful speech signals in noise have not been successful so far.

In recent years, research has progressed on analyzing the brain during speech production with a high-resolution device such as PET and fMRI and monitoring ECoG when a patient speaks at the time of craniotomy, and it is becoming clearer which part of the brain processes speech. According to the results, after concept preparation in the left middle temporal gyrus (MTG), planning for speech takes place in the left superior temporal gyrus (STG) (see Non-Patent Document 2). This is followed by syllabication in the left inferior frontal gyrus (IFG; Broca's area), and articulation occurs in the left precentral gyrus (PG; motor area) while speech is produced (see Non-Patent Document 3). Based on these research findings, it is expected that decoding silent or imagined speech is enabled if the linguistic representation that reaches Broca's area can be captured.

In addition, there has been proposed a technology in which brain waves are detected to extract signals related to a motor command from the brain waves (see Patent Document 1) .

[Prior Art Document]

[Non-Patent Document]

Non-Patent Document 1: Heger D. et al., Continuous Speech Recognition from ECoG, Interspeech2015, 1131-1135 (2015)
Non-Patent Document 2: Indefrey, P et al., The spatial and temporal signatures of word production components, Cognition 92, 101-144 (2004)
Non-Patent Document 3: Bouchard K.E. et al., Functional organization of human sensorimotor cortex for speech articulation, Nature 495, 327-332 (2013)
Non-Patent Document 4: Girolami M., Advances in Independent Component Analysis, Springer (2000)
Non-Patent Document 5: Durbin, J. "The fitting of time series models." Rev. Inst. Int. Stat., v. 28, pp. 233-243 (1960)

[Patent Document]

Patent Document 1: Japanese Unexamined Patent Application Publication No. 2008-204135

[Summary of the Invention]

[Problems to be Solved by the Invention]

The biggest problem in speech recognition from EEG signals is that since it is unclear in what format the linguistic representation is expressed, a specific extraction method cannot be found out. Furthermore, without a method for converting the linguistic representation to phoneme units, efficient speech processing is hardly feasible because of many types of targets such as syllabic units. There are said to be thousands of syllables including many long syllables as well as short syllables; there are about 24 phonemes in Japanese and 44 phonemes in English (English vowels are classified into tense and lax vowels, while generally Japanese vowels are not).

The present invention has been made in view of the above problems. An object of the present invention is to provide a speech imagery recognition device, a wearing fixture, a speech imagery recognition method, and a program that enable speech recognition using EEG signals.

[Means for Solving the Problems]

To achieve the object mentioned above, the present invention is mainly characterized in that, in order to recognize speech from EEG signals during speech imagery, line spectral components are extracted as a linguistic representation by a line spectral component extractor, and these components are passed through a phoneme-feature vector time sequence converter that uses phoneme-specific convolution operation or the like to thereby obtain a phoneme-feature vector time sequence.

According to the first aspect of the present invention, there is provided a speech imagery recognition device that recognizes speech from EEG signals during speech imagery. The speech imagery recognition device includes: an analysis processor that analyzes discrete signals, which are obtained from EEG signals received from a plurality of electrodes, for each of the electrodes and outputs a spectral time sequence; and an extractor that outputs a phoneme-feature vector time sequence based on the spectral time sequence.

According to the second aspect of the present invention, there is provided a wearing fixture for a speech imagery recognition device that recognizes speech from EEG signals during speech imagery. The wearing fixture includes a plurality of electrodes configured to be placed over Broca's area and an output unit that outputs signals from the electrodes. The speech imagery recognition device is configured to: analyze discrete signals, which are obtained from EEG signals output from the output unit, for each of the electrodes to output a spectral time sequence; and extract and output a phoneme-feature vector time sequence based on the spectral time sequence.

According to the third aspect of the present invention, there is provided a speech imagery recognition method for recognizing speech from EEG signals during speech imagery. The speech imagery recognition method includes: analyzing discrete signals, which are obtained from EEG signals received from a plurality of electrodes, for each of the electrodes to output a spectral time sequence; and extracting and outputting a phoneme-feature vector time sequence based on the spectral time sequence.

According to the fourth aspect of the present invention, there is provided a program that causes a computer to perform a speech imagery recognition process of recognizing speech from EEG signals during speech imagery. The program causes the computer to: analyze discrete signals, which are obtained from EEG signals received from a plurality of electrodes, for each of the electrodes to output a spectral component as a linguistic representation; and extract phoneme features based on the spectral component for each of the electrodes.

[Effects of the Invention]

According to one aspect of the present invention, it is possible to provide a speech imagery recognition device, a wearing fixture, a speech imagery recognition method, and a program that enable speech recognition using EEG signals.

[Brief Description of the Drawings]

[FIG. 1] FIG. 1 is a model diagram illustrating the configuration of a recognition device according to an embodiment of the present invention.
[FIG. 2] FIG. 2 is a diagram illustrating electroencephalography (EEG) electrodes (10-10 system) and 9 electrodes placed over Broca's area.
[FIG. 3] FIG. 3 is a diagram illustrating the effect of noise removal from EEG signals.
[FIG. 4] FIG. 4 is a diagram for explaining the linear predictive analysis of EEG signals during speech imagery.
[FIG. 5] FIG. 5 is a diagram illustrating EEG recordings during speech imagery in a comparison between the linear predictive analysis and the conventional Fourier analysis.
[FIG. 6] FIG. 6 is a diagram illustrating short sine waves of EEG during speech imagery.
[FIG. 7] FIG. 7 is a flowchart illustrating the operation of a linguistic feature extractor.
[FIG. 8] FIG. 8 is a diagram illustrating an example of absorption of frequency fluctuations in EEG during speech imagery.

[FIG. 9] FIG. 9 is a diagram illustrating an example of a line spectral time sequence of EEG signals during speech imagery.

[FIG. 10] FIG. 10 is a diagram illustrating an example of a line spectral time sequence straddling a plurality of electrodes.

[FIG. 11] FIG. 11 is a flowchart illustrating a processing procedure for designing and using a phoneme-specific convolution operator.

[FIG. 12] FIG. 12 is a diagram illustrating an example of phoneme eigenvectors constituting a phoneme-specific convolution operator.

[FIG. 13] FIG. 13 is a diagram illustrating an example of a phoneme likelihood time sequence for EEG signals during speech imagery.

[FIG. 14] FIG. 14 is a diagram illustrating electrode position correction by test recognition.

[FIG. 15] FIG. 15 is a diagram illustrating another example of the configuration of a speech imagery recognition device.

[FIG. 16] FIG. 16 is a diagram illustrating another example of the configuration of a speech imagery recognition device.

[FIG. 17] FIG. 17 is a diagram illustrating another example of the configuration of a speech imagery recognition device.

[Modes for Carrying Out the Invention]

(Embodiments)

In the following, exemplary embodiments of a speech imagery recognition device according to the present invention will be described with reference to the accompanying drawings. Note that the drawings are used to illustrate the technical features of the invention, and are not intended to limit the configuration of the device as well as various processing procedures and the like to those aspects illustrated therein unless otherwise specifically mentioned. Incidentally, like parts are designated by like reference numerals or characters throughout the description of the embodiments.

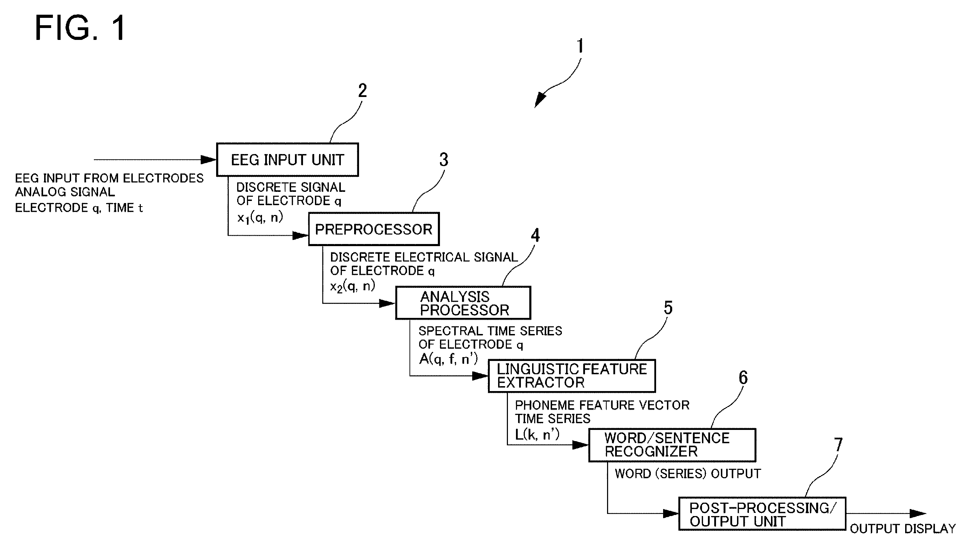

FIG. 1 is a model diagram illustrating the configuration of a speech imagery recognition device 1. The configuration and operation of the speech imagery recognition device 1 will be described with reference to FIG. 1. The speech imagery recognition device 1 is used to recognize speech or spoken language from electroencephalogram (EEG) signals during speech imagery.

The speech imagery recognition device 1 includes an EEG input unit 2, a preprocessor 3, an analysis processor 4, a linguistic feature extractor 5, a word/sentence recognizer 6, and a post-processing/output unit 7. The EEG input unit 2 is configured to convert EEG signals received from a plurality of electrodes placed on the scalp (not illustrated) into discrete signals. The preprocessor 3 is configured to remove noise from the discrete signals for each electrode. The analysis processor 4 is configured to analyze the discrete signals for each electrode and output a spectral time sequence. The linguistic feature extractor 5 is configured to extract and output a phoneme-feature vector time sequence based on the spectral time sequence of all the electrodes. The word/sentence recognizer 6 is configured to recognize words and sentences that constitute a spoken language from the phoneme-feature vector time sequence. The post-processing/output unit 7 is configured to display speech information or output the information in audio.

The EEG input unit 2 converts analog signals $x(q, t)$ output from the EEG electrodes into discrete signals through A/D conversion or the like, and corrects the bias of the individual electrodes by using the average value of discrete signals of all the electrodes or the like. At the same time, the EEG input unit 2 removes unnecessary frequency components of 70 Hz or below by a low-frequency removal filter (highpass filter) and unnecessary frequency components of 180 Hz or above by a high-frequency removal filter (low-pass filter) from discrete signals for each electrode, thereby outputting a signal $x_1(q, n)$.

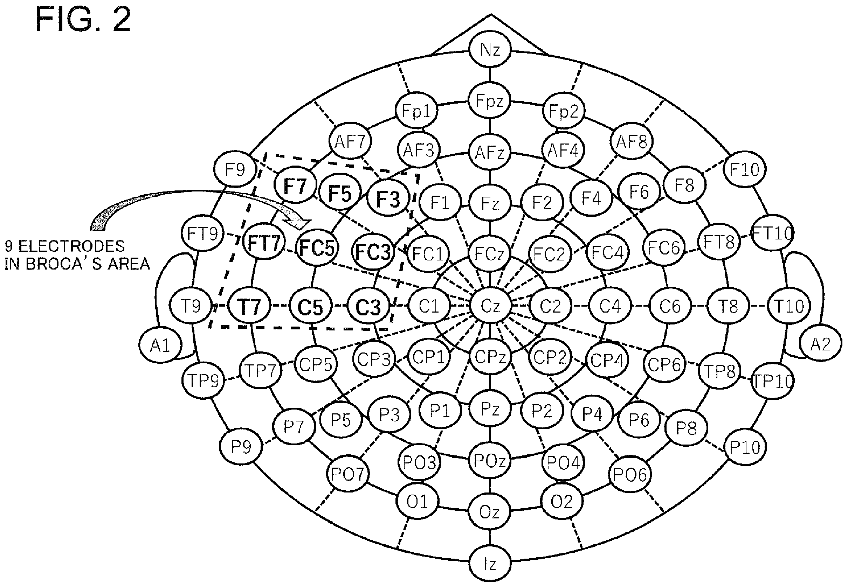

FIG. 2 illustrates the layout of 64 electrodes according to the standard international 10-10 system. Of these electrodes, speech imagery signals are received from 9 electrodes (F3, F5, F7, FC3, FC5, FT7, C3, C5, T7) placed over Broca's area of the left brain, and linguistic features are extracted to recognize imagery contents. It is generally said that right-handed people process language in the left brain and a significant number of left-handed people also process language in the left brain. Incidentally, although EEG signals may be affected by large noises (called artifacts) due to movements such as blinking, many unnecessary components can be removed by the above filtering process. Further, to clean unnecessary components that cannot be removed by the filtering process, independent component analysis (ICA) may be applied to discrete signals of all the electrodes, where after a small number of independent information sources are estimated and removed, the original output of the electrodes (9 electrodes in this case) is recovered.

The preprocessor 3 removes noise that has passed through the filters for each electrode. One example of this process will be described below. First, the discrete signal $x_1(q, n)$ (q: electrode number, n: time) of each electrode, which has undergone a series of processes in the EEG input unit 2, is multiplied by a certain time window, and then it is mapped from the time domain to the frequency domain using the Fast Fourier Transform (FFT). Thereafter, an amplitude spectral time sequence $X_1(q, f, n')$ (f: frequency, n': time frame number after windowing) is obtained from complex number components in the frequency domain as follows:
[Formula 1]

$$\mathrm{FFT} : \quad x_1(q, \ n) \ \rightarrow \ \mathrm{Re}\{X_1(q, \ f, \ n')\} \ + \ j \ \mathrm{Im}\{X_1(q, \ f, \ n')\} \qquad (1)$$

[Formula 2]

$$X_1(q, \ f, \ n') = [\mathrm{Re}\{X_1(q, \ f, \ n')\}^2 \ + \ \mathrm{Im}\{X_1(q, \ f, \ n')\}^2]^{1/2} \qquad (2)$$

where j represents an imaginary unit, and Re{} and Im{} represent a real part and an imaginary part, respectively. In noise subtraction, an average noise amplitude spectrum is obtained from the spectrum N(q, f, n') of an EEG signal recorded prior to speech imagery by the following formula.
[Formula 3]

$$N_{av}(q, f, n') = (\frac{1}{17}) \sum_{n=n'-8}^{n'+8} N(q, f, n') \qquad (3)$$

In the above formula, an average noise spectrum is calculated from 8 frames before and after time n'; however, it may be set as appropriate depending on the system. In the setting of time n', generally, there may be the following two ways:

(a) The user performs speech imagery in response to a prompt signal (a signal that indicates the start of imagery) provided by a speech imagery recognition application system.
(b) The user performs speech imagery after providing the application system with a predetermined call (wake-up word) such as "Yamada-san".

In both cases, N(q, f, n') is calculated from EEG signals recorded in the section before or after the speech imagery.
Then, for each electrode q, Nav(q, f, n') is subtracted from the speech imagery signal spectrum $X_1$(q, f, n') as represented by the following formula:
[Formula 4]

$$X_2(q, f, n') = X_1(q, f, n') \ - \ Nav(q, f, n') \qquad (4)$$

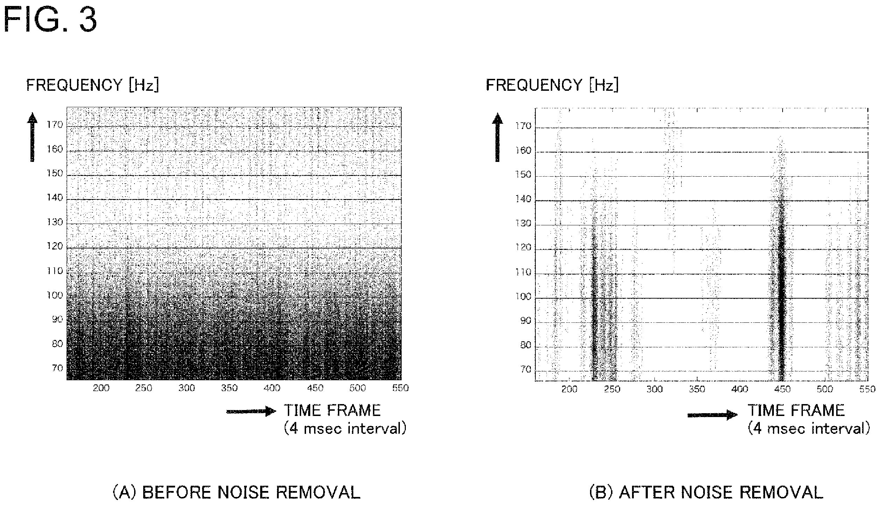

FIG. 3 illustrates an example in which noise is removed from EEG signals by this process; FIG. 3(A) illustrates EEG before noise removal, and FIG. 3(B) illustrates EEG after noise removal. It can be seen from comparing FIGS. 3(A) and 3(B) that the effect on removing the noise spectrum is remarkable. The waveform $x_2$(q, n) is recovered from the amplitude spectral time sequence after noise removal by the inverse fast Fourier transform (IFFT).
It should be noted that it is effective to perform the process of extracting a small number of independent information sources from signals of the 9 electrodes after noise removal, i.e., independent component analysis (ICA) (Non-Patent Document 4). This process can remove unnecessary components that cannot be removed by the filtering process and can also select a small number of effective information sources from discrete signals of the 9 electrodes. However, ICA has a drawback of so-called permutation in which the order of independent components varies in the result of each analysis. How this drawback is eliminated to incorporate ICA into this embodiment will be explained later.
While the analysis processor 4 may use the spectral time sequence $x_2$(q, f, n') of the speech imagery signal after noise removal (and after extraction of q independent components) obtained by the preprocessor 3, linear predictive analysis (LPA) is used as an analysis method that brings out better the effect of the present invention in an example described below. The analysis processor 4 can use a spectrum or a line spectrum.
Linear predictive coding (LPC) is currently a global standard method for speech communication. There are two information sources in speech: pulse waves at a constant frequency produced by the vocal cords and random waves produced by narrowing the vocal tract. For this reason, a complex process is required where sound sources are stored separately as a codebook, all the sound sources in the codebook are passed through the linear prediction coefficient of speech (which is responsible for the transfer function of the vocal tract), and then the synthesized speech is compared with the original speech.

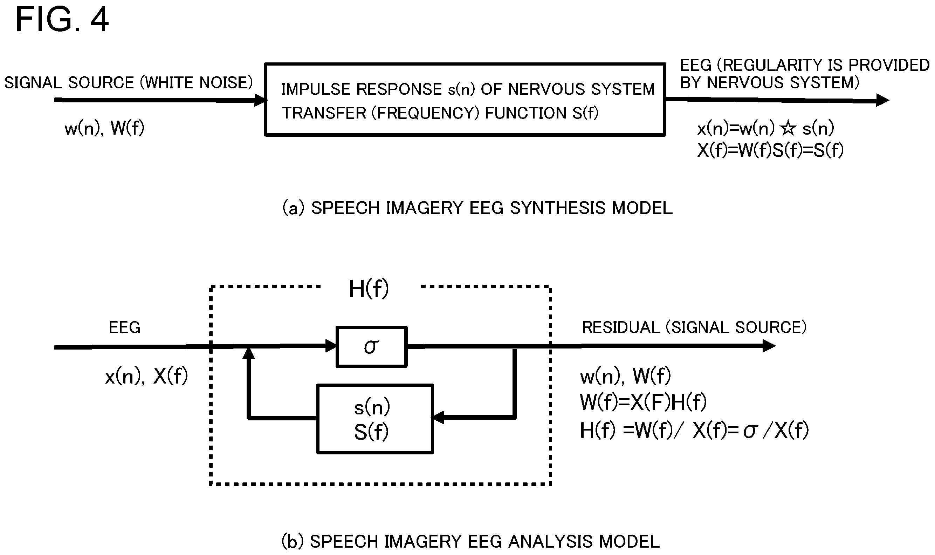

On the other hand, the only source of information in brain waves is considered to be random waves as illustrated in FIG. 4, and therefore EEG synthesis is simpler than speech synthesis. There are various algorithms, such as the Levinson-Durbin method, that are available to obtain the linear prediction coefficient $\{\alpha_m\}$ from the autocorrelation coefficient $r_2(\tau)$ obtained from an EEG signal $x_2(q, n)$ (Non-Patent Document 4). As illustrated in FIG. 4, the white noise $w(n)$ of the signal source is passed through the impulse response $s(n)$ of the nervous system to obtain each electrode's EEG signal $x(n)$ during speech imagery. In FIG. 4, ☆ represents the convolution integral symbol.

In the convolutional integration, EEG spectrum can be expressed as $X(f) = W(f)S(f) = S(f)$ (note: $W(f) = 1$), where $S(f)$ represents the transfer (frequency) function of the impulse response $s(n)$, which is responsible for spoken language information in the frequency domain. The function $S(f)$ can be obtained from the Fourier transform of the linear prediction coefficient $\{\alpha_m\}$ as represented by the following formula:
[Formula 5]

$$X(f) = S(f) = \mathscr{F}[s(n)] = \mathscr{F}[\alpha_0 \delta(n) + \alpha_1 \delta(n-1) + \alpha_2 \delta(n-2) + \cdots + \alpha_p \delta(n-p)] \qquad (5)$$

where $\delta(n-p)$ is a function that represents the time $n = p$ of a signal, and $F[]$ is the Fourier transform. In linear predictive analysis (LPA) for EEG signals, as illustrated in FIG. 4, the following formula can be obtained using the synthesis model $S(f)$ as an inverse filter:
[Formula 6]

$$H(f) = \sigma / X(f) = \sigma / \mathscr{F}[\alpha_0 \delta(n) + \alpha_1 \delta(n-1) + \alpha_2 \delta(n-2) + \cdots + \alpha_p \delta(n-p)] \qquad (6)$$

where $\sigma$ is an amplitude bias value. This method of performing accurate analysis throughout the synthesis process is called "Analysis-by-Synthesis (AbS)" and is also effective in EEG analysis. In the Fourier transform $F[]$ of the above formula, 0 points are added to p linear prediction coefficients ($\alpha_0 = 1.0$) (called zero padding), which enables the Fourier transform of any number of points such as, for example, 128 points, 256 points, .... With the zero padding, the frequency resolution accuracy can be arbitrarily adjusted to 64 points, 128 points, ... to obtain a spectral component $A(q, f, n')$.

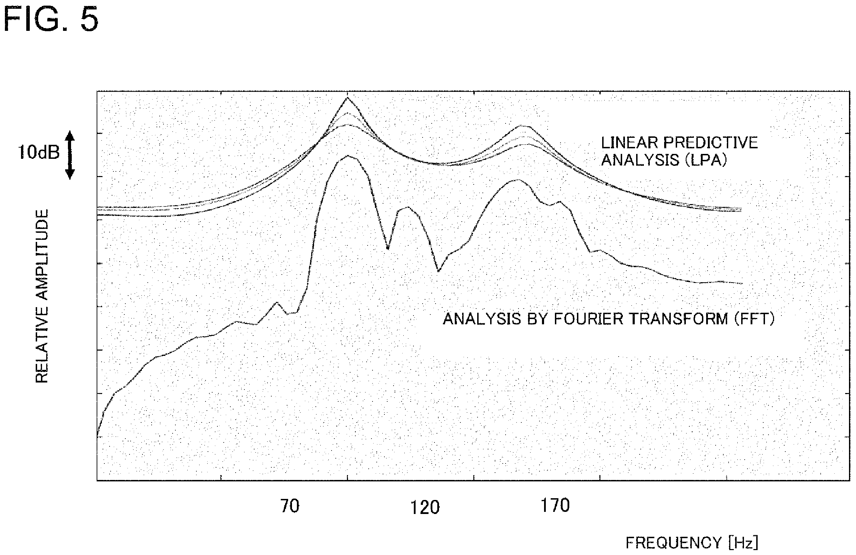

FIG. 5 illustrates a spectral pattern analyzed by LPA in comparison with a spectral pattern analyzed by the ordinary Fourier transform. In FIG. 5, there is an illustration of a plurality of spectral patterns obtained by LPA. This indicates the use of a window function called log window that attenuates the value as the delay $\tau$ increases with respect to the autocorrelation coefficient (the top one is obtained with no lag window, the slope of the lag window is greater toward the bottom, and the peak is sharp when no log window is used). In LPA, as illustrated in FIG. 5, the spectrum can be represented with a small number of essential peaks present in EEG signals.

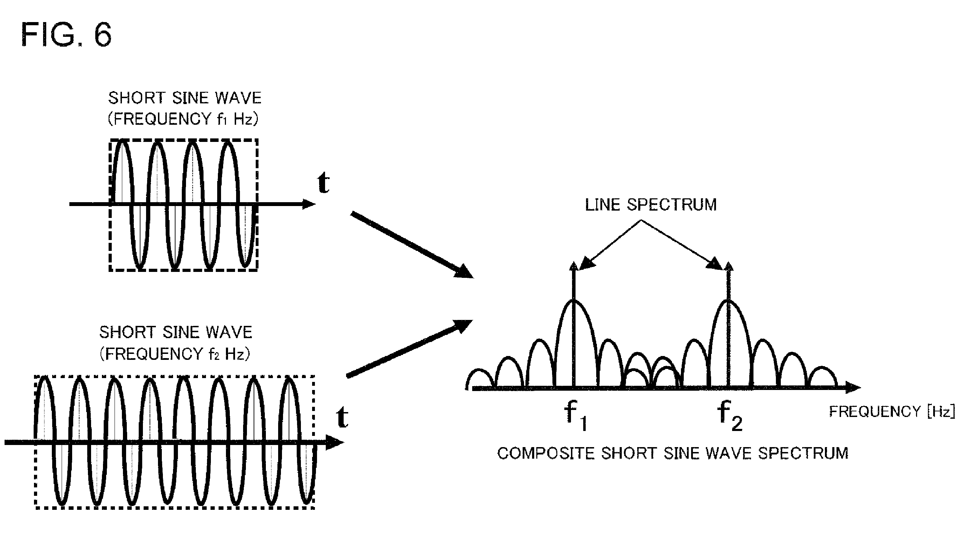

Through the LPA analysis, the spectrum of EEG during speech imagery is represented with a small number of spectral peaks. This suggests that in the brain (especially in Broca's area where linguistic information of speech imagery is produced), the linguistic representation is composed of short sine waves (tone burst), in other words, the linguistic representation is represented by a unique line spectrum. FIG. 6 illustrates an example of tone burst waves and their spectral shape. A short sine wave is supposed to be represented by a single parameter, i.e., a single frequency; however, as illustrated in FIG. 6 (and FIG. 5), transients at the beginning and end of the signal cause a spread in the spectrum in general frequency analysis.

The linguistic feature extractor 5 extracts line spectral components as "linguistic representation" from spectra with a spread and outputs a phoneme-likelihood vector time sequence, which is a linguistic feature, through a phoneme-specific convolution operator.

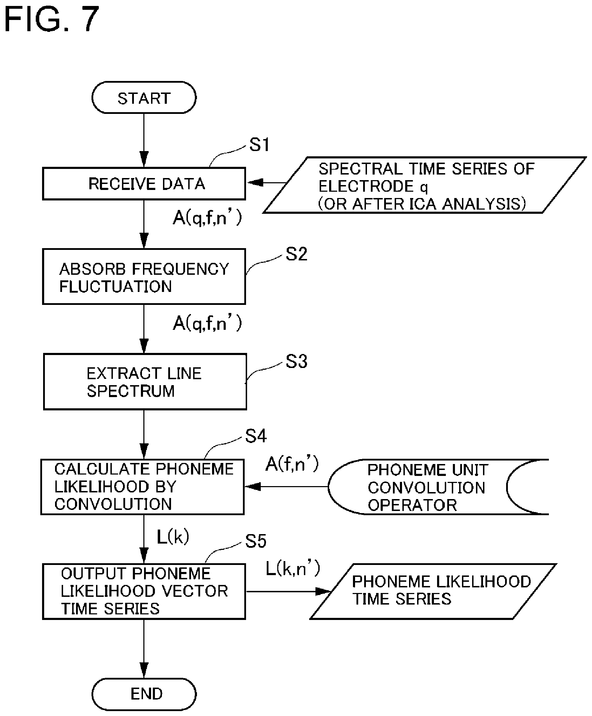

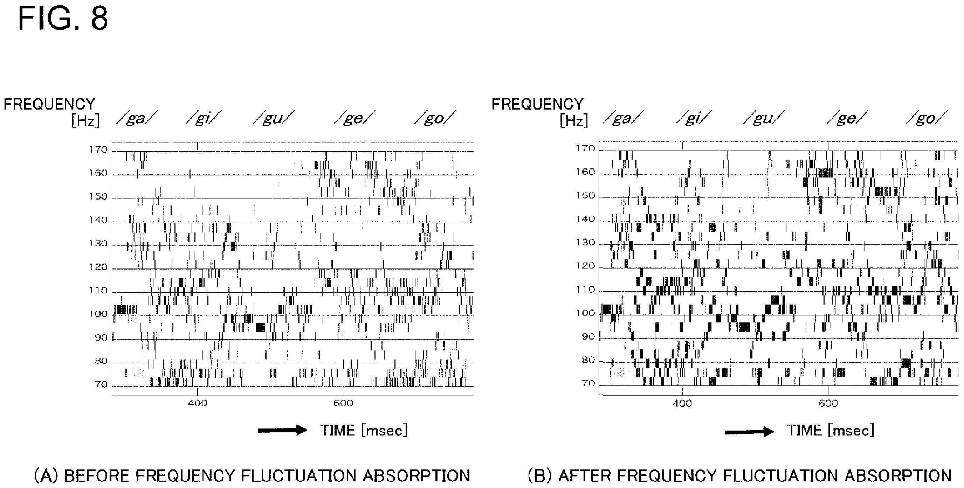

The processing procedure will be described below with reference to the flowchart of FIG. 7 illustrating the operation of the linguistic feature extractor 5. First, the linguistic feature extractor 5 receives a spectral time sequence of electrode q from the analysis processor 4 (step S1). The spectrum of EEG during speech imagery may have a fluctuation of about $\pm 5$ Hz as illustrated in FIG. 8(A). Therefore, these frequency fluctuations are absorbed using a median filter, a type of nonlinear filtering (step S2).

For data within a certain time width (several frames before and after time n') and a frequency width (adjacent frequencies f-1, f, f+1), the intermediate value of the whole is obtained and used as a representative value. This process can absorb frequency fluctuations as it can remove values deviating from the median value. The output of the nonlinear filter is generally smoothed by a Gaussian window or the like. FIG. 8(B) illustrates the result of intermediate value filtering applied to a total of 7 frames (the center frame n' and 3 frames before and after it) for EEG signals of 70 Hz to 170 Hz (4 msec period) to improve the frequency fluctuation. It can be seen from the figure that the fluctuation is reduced. After that, the frequency analysis pattern is smoothed by multiplying it by a Gaussian window (coefficient; {1/4, 1/2, 1/4}) in the time direction, and time frames are dropped from 4 msec to around 8 msec. The process of absorbing frequency fluctuations can also be performed in the preprocessor 3 after subtracting noise components on the amplitude spectrum and before recovering the waveform signal.

Next, the process of extracting a line spectrum (step S3) will be described. In this process, components derived from the peak that appears on the frequency axis is extracted as a line spectrum for each time frame (8 msec) . Specifically, only the frequencies that satisfy the following conditions are defined as sinusoidal frequency components with the original amplitude, i.e., line spectrum components:

(i) Frequency at which the maximum value $\Delta_f = 0$ on the frequency axis

(ii) When the inflection point $\Delta\Delta_f = 0$

if $\Delta_f > 0$, frequency at which the value of $\Delta\Delta_f$ changes from positive to negative
if $\Delta_f < 0$, frequency at which the value of $\Delta\Delta_f$ changes from negative to positive

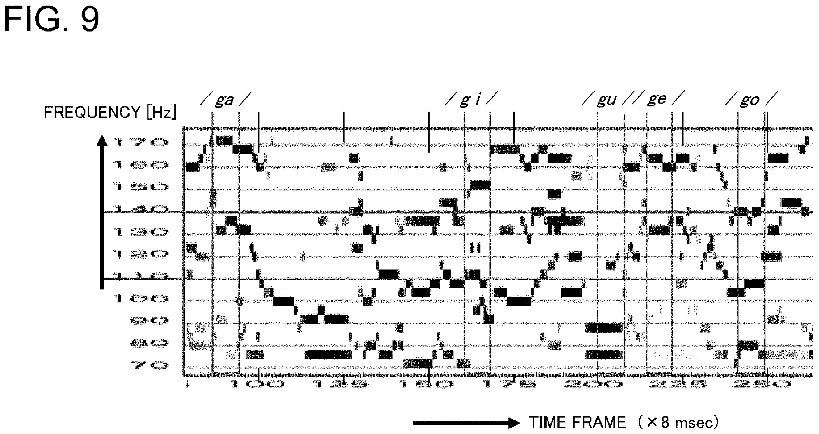

FIG. 9 illustrates an example of extracted line spectral components of EEG during speech imagery. In this example, data is collected under the task of imagining /ga-gi-gu-ge-go/ three times in succession as much as possible. By repeating the same sequence three times, a skilled person can learn the pattern of each syllable as illustrated in the figure, and can create a database of EEG data with syllable labels.

FIG. 9 illustrates the result of syllable labeling applied to the integrated line spectra after pooling of line spectral time sequences of 9 electrodes in the direction of the electrodes (extracting a representative pattern from the 9 electrodes, e.g., taking the p-norm (p = ∞ corresponds to taking the maximum value)). In this example, the pooling process is performed only for reading the syllable labels, and the following phoneme feature extraction is carried out on the original line spectral components of the 9 electrodes.

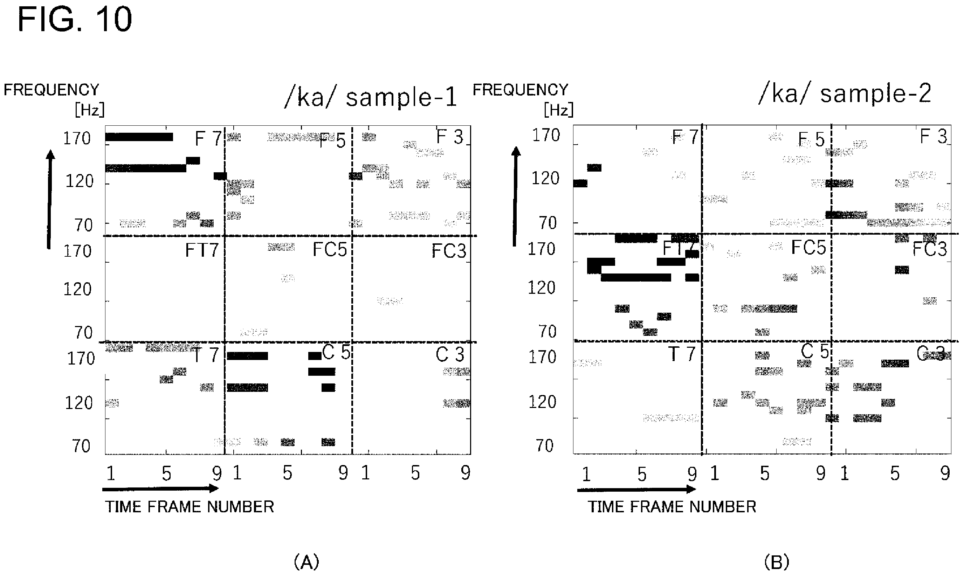

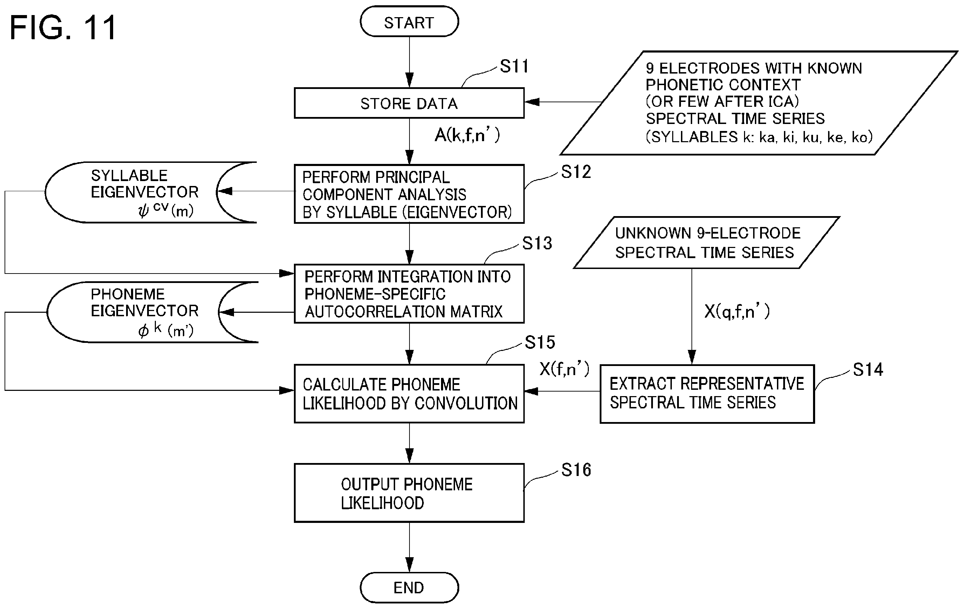

The linguistic feature extractor 5 is aimed at extracting phoneme features in the end. Specifically, it is aimed at extracting phoneme components, which are the smallest unit of speech information, in the form of a phoneme feature vector from line spectral components of each electrode. Speech information in EEG signals has the so-called tensor structure that spans three axes: line spectrum (frequency information), electrodes (spatial information), and frames (temporal information). FIG. 10 illustrates an example of a line spectral time sequence over $3 \times 3 = 9$ electrodes in Broca's area. The figure illustrates the case of a monosyllable /ka/ as an example. As illustrated, the syllable pattern that occurs in Broca's area varies in electrode position each time it occurs, which suggests the flexible information processing mechanism of the cranial nerve system. Meanwhile, in the speech processing of the brain, syllables appear in Broca's area as the smallest unit of speech. During speech production, the vocal organs are controlled by muscle movements, and this control is performed with articulatory parameters that correspond one-to-one to phonemes. Given this background, there is likely a process of extracting phoneme features from the syllable pattern of FIG. 10 observed in Broca's area. A method of realizing this process on a computer will be described below with reference to the flowchart of FIG. 11 illustrating a processing procedure for designing and using a phoneme-specific convolution operator.

The flowchart of FIG. 11 illustrates the calculation of a phoneme likelihood vector by a phoneme-specific convolution operator for efficiently extracting phonemes from the frequency-time pattern of 9 electrodes. First, syllables belonging to the same phonemic context (for the phoneme /s/: /sa/, /shi/, /su/, /se/, /so/; for the phoneme /a/: /a/, /ka/, /sa/, /ta/, /na/, /ha/, ..., /ga/, /za/, ..., etc.) are stored in a memory (step S11). The method in which this stored information is retrieved for use in necessary information processing and returned after finishing is called pooling.

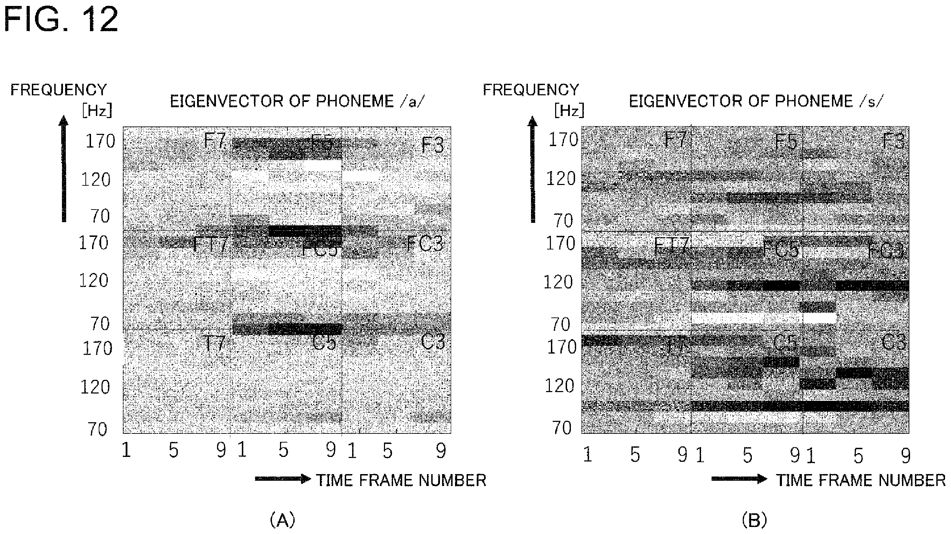

Next, principal component analysis is performed for each syllable (step S12), and eigenvectors for each syllable are phoneme-grouped with respect to each relevant phoneme in a manner as follows: the phoneme /s/: $\{\psi^{/sa/}(m), \psi^{/shi/}(m), \psi^{/su/}(m), \psi^{/se/}(m), \psi^{/so/}(m)\}$, the phoneme /a/: $\{\psi^{/a/}(m), \psi^{/ka/}(m), \psi^{/sa/}(m), \psi^{/ta/}(m), \psi^{/na/}(m), ....,\}$. Then, the autocorrelation matrix is calculated from the eigenvectors of the same phoneme group and integrated into a phoneme-specific autocorrelation matrix $R^s$, $R^a$, ... (step S13). From the phoneme-specific autocorrelation matrix, subspaces (eigenvectors) $\varphi^{/s/}(m)$, $\varphi^{/a/}(m)$ for respective phonemes can be obtained. FIG. 12 illustrates the eigenvectors of the phonemes /s/ and /a/ (representing the accumulation of the top three axes).

After that, by using eigenvectors obtained for each phoneme k as "phoneme-specific convolution operator", the phoneme similarity (likelihood) L(k) is calculated for unknown 9-electrode (or a few after ICA) line spectral time sequences (step S4, step S14, step S15).

[Formula 7]

$$L(k) = {}^{Max}_{q} < X(q, f, n'), \phi(f, n') >^2 \quad , k = 1, 2, .., K \quad (7)$$

where Max means to take the maximum value for q electrodes or q ICA components, and <> represents an inner product operation. Note that X(q, f, n') and $\varphi$(f, n') are each normalized by a norm in advance.

A phoneme feature vector is defined as a vector composed of a series of K likelihoods $L(k)$ of phoneme k; k = 1, 2, ..., K. In formula (7), the eigenvector $\varphi(f, n')$ of the phoneme is used to construct the phoneme-specific convolution operator, and a scalar value $L(k)$ is obtained for each phoneme k as a likelihood. A vector of K scalar values is output from the linguistic feature extractor 5 as (phoneme-likelihood vector) time-sequence data as the time n' of input $X(f, n')$ advances (step S5, step S16).

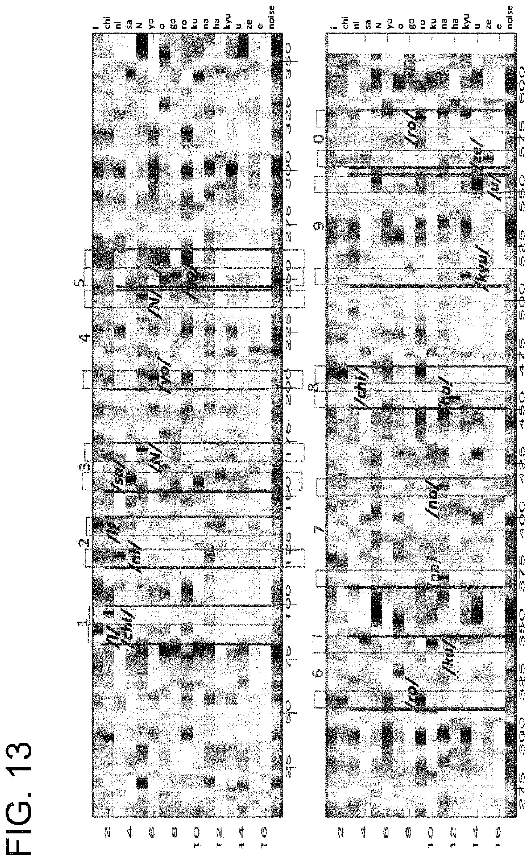

FIG. 13 illustrates an example in which the likelihood of syllables $(L(go), L(ro), ...)$ is obtained from the likelihood of phonemes $(L(g), L(o), ...)$. In this example, the shades indicate the likelihood of syllables when consecutive numbers ("1, 2, 3, 4, 5, 6, 7, 8, 9, 0") are imagined in this order, and the syllables (i, chi, ni, sa, N, yo, o, go, ro, ku, na, ha, kyu, u, ze, e, noise) are represented on the vertical axis. It can be seen that the likelihood of the syllables that constitute the consecutive numbers is obtained with a high value.

Since it is difficult to collect a large amount of speech imagery data at present, the problem is solved through a phoneme convolution operator in the example described herein. However, as the brain database related to speech imagery becomes more complete in the future, it will be possible to use a deep convolutional network (DCN), which have been widely used in such fields as image processing in recent years, instead of the phoneme-specific convolution operator.

The word/sentence recognizer 6 recognizes a word/sentence from the time-sequence data of the phoneme feature vector (more specifically, phoneme-likelihood vector time-sequence data). There are some methods that can be applied to word/sentence recognition such as a method that uses a hidden Markov model (HMM) (where a triphone including a sequence of three consecutive phonemes is used), which has been put to practical use in the field of speech recognition, and a method that uses a deep neural network (LSTM, etc.). In addition, linguistic information (probability as to word sequence), an advantage of current speech recognition, can be used as well. Furthermore, as the time axis shift is a concern in speech imagery, the use of "spotting", which is performed in the current robust audio system to continuously search for words and sentences in the time direction, is also effective in improving quality in speech imagery.

The post-processing/output unit 7 receives a word (sequence) of the recognition result and produces a required display and audio output. Here, the post-processing/output unit 7 may have a function of providing the user with feedback on whether the multi-electrode EEG sensor is in the correct position based on the result of speech imagery recognition of a predetermined word/sentence so that the user can move the EEG sensor through the screen of a terminal such as a smartphone or a voice instruction, thereby helping the user to find the proper position.

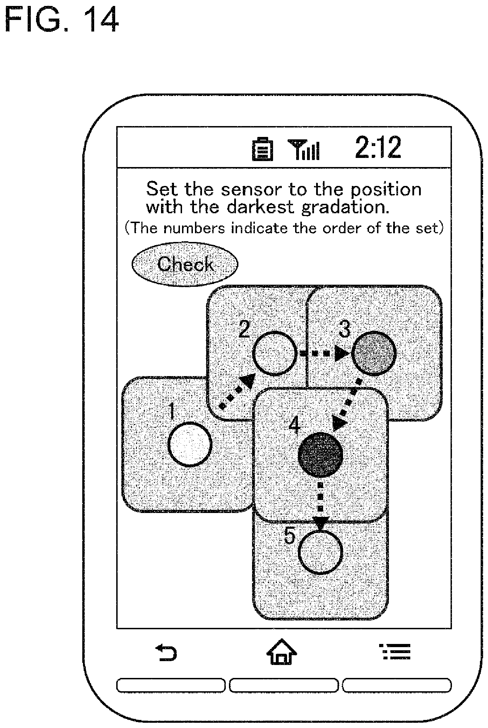

The post-processing/output unit 7 displays a screen that helps the user adjust the optimal position of the electrodes while performing speech imagery. FIG. 14 illustrates an example of a display screen that may be provided by the post-processing/output unit 7. The user adjusts the positions of the electrodes while looking at the screen illustrated in FIG. 14.

As illustrated in FIG. 14, when the user imagines speech (such as "Yamada-san") as test speech imagery, EEG signals are received through the EEG input unit 2, and the accuracy of the recognition result can be indicated on the screen displayed by the post-processing/output unit 7 with color, size of O, gradation (the example of FIG. 14), or the like. In FIG. 14, the first electrode position (1) is displayed in white, the next electrode position (2) is displayed in light gray, the electrode position (3) is displayed in gray, the electrode position (4) is displayed in dark gray, and the electrode position (5) is displayed in light gray. This allows the user to know that the electrode position (4) is the optimal position. Described above is an example where the post-processing/output unit 7 has a function that prompts the user to move the sensor position in the proper direction to correct it while viewing the difference in accuracy in chronological order.

The speech imagery recognition device 1 illustrated in FIG. 1 can comprise a mobile terminal. The speech imagery recognition device 1 can also comprise a server. In this case, the speech imagery recognition device 1 may include a plurality of servers. Furthermore, the speech imagery recognition device 1 can comprise a mobile terminal and a server so that part of its processing can be performed by the mobile terminal and the rest by the server. In this case also, there may be a plurality of servers.

While the speech imagery recognition device 1 has been described as including the EEG input unit 2, the preprocessor 3, the analysis processor 4, the linguistic feature extractor 5, the word/sentence recognizer 6, and the post-processing/output unit 7 as illustrated in FIG. 1, it may further include a wearing fixture and electrodes.

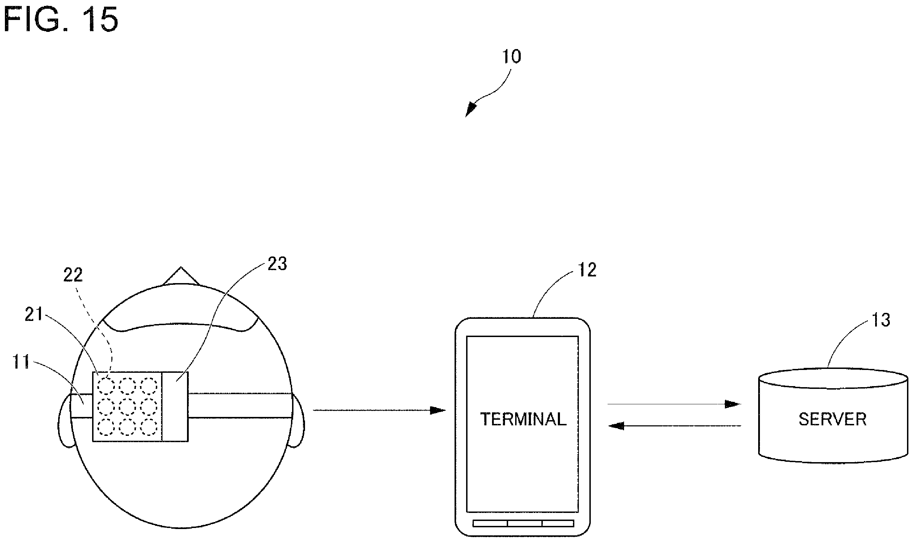

FIG. 15 is a diagram illustrating another example of the configuration of a speech imagery recognition device. As illustrated in FIG. 15, a speech imagery recognition device 10 includes a wearing fixture 11, a mobile terminal 12, and a server 13. The wearing fixture 11 is used for the speech imagery recognition device that recognizes speech from EEG signals during speech imagery. The wearing fixture 11 includes a sheet 21 that holds an electrode set 22, the electrode set 22 configured to be placed over Broca's area, and a processor 23 configured to output signals from the electrode set 22. Although the electrode set 22 is composed of 9 electrodes in this embodiment as described above, the number of electrodes is not particularly limited. The processor 23 may have a communication function, and it can perform part or all of the processing of the speech imagery recognition device 1 illustrated in FIG. 1.

The processor 23 of the wearing fixture 11, the mobile terminal 12, and the server 13 comprise, for example, a computer that includes a central processing unit (CPU), a memory, a read-only memory (ROM), a hard disk, and the like. The mobile terminal 12 can perform part or all of the processing of the speech imagery recognition device 1 illustrated in FIG. 1. The server 13 can also perform part or all of the processing of the speech imagery recognition device 1 illustrated in FIG. 1.

A speech imagery recognition method of recognizing speech from EEG signals during speech imagery is performed by the wearing fixture 11, the mobile terminal 12, and/or the server 13; the wearing fixture 11, the mobile terminal 12, and/or the server 13 can perform the method independently or in collaboration with one another. The speech imagery recognition method can be performed by the mobile terminal 12 and the server 13.

A program that causes a computer to perform a speech imagery recognition process of recognizing speech from EEG signals during speech imagery may be downloaded or stored in the hard disk or the like. The program causes the computer to perform the analysis process of analyzing discrete signals of EEG signals received from a plurality of electrodes for each electrode and outputting a spectral time sequence, and the extraction process of extracting a phoneme-feature vector time sequence based on spectral components of each electrode.

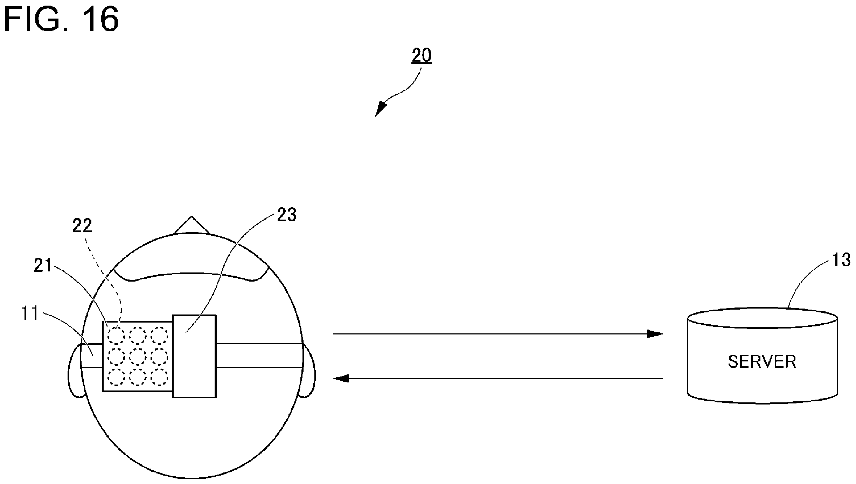

FIG. 16 is a diagram illustrating another example of the configuration of a speech imagery recognition device. As illustrated in FIG. 16, a speech imagery recognition device 20 includes the wearing fixture 11 and the server 13. While the wearing fixture 11 is configured as described above with reference to FIG. 15, the processor 23 thereof has a function of directly communicating with the server 13. Thus, the wearing fixture 11 directly exchanges information with the server 13, thereby realizing the function of the speech imagery recognition device.

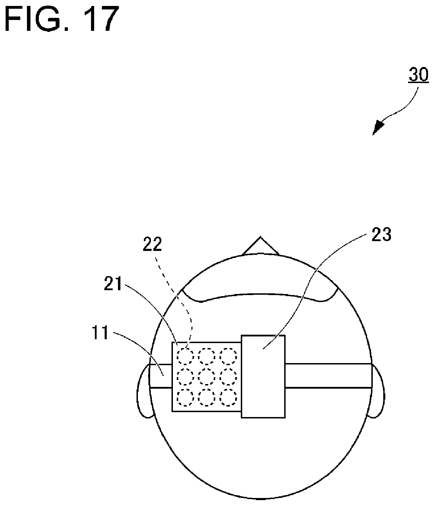

FIG. 17 is a diagram illustrating another example of the configuration of a speech imagery recognition device. As illustrated in FIG. 17, a speech imagery recognition device 30 includes the wearing fixture 11. The processor 23 of the wearing fixture 11 implements all the functions of the speech imagery recognition device illustrated in FIG. 1. As a result, the speech imagery recognition device 30 can be realized only by the wearing fixture 11.

As described above, according to the embodiments, line spectral components as a linguistic representation can be directly extracted from EEG signals during speech imagery and converted into a phoneme-feature vector time sequence. This offers the advantage that the current framework of speech recognition can be utilized.

In the following, additional notes will be provided with respect to the above embodiments.

(Additional note 1)

A speech imagery recognition method for recognizing speech from electroencephalogram (EEG) signals during speech imagery, the method comprising:

an analysis process of analyzing discrete signals, which are obtained from EEG signals received from a plurality of electrodes, for each of the electrodes and outputting a spectral time sequence; and

an extraction process of outputting a phoneme-feature vector time sequence based on the spectral time sequence.

(Additional note 2)

The speech imagery recognition method as set forth in additional note 1, further comprising converting the EEG signals received from the electrodes to the discrete signals.

(Additional note 3)

The speech imagery recognition method as set forth in additional note 1 or 2, further comprising preprocessing of subtracting an average noise amplitude spectrum from a spectrum of a speech imagery signal obtained by converting the discrete signals to a frequency domain to remove noise from the EEG signals.

(Additional note 4)

The speech imagery recognition method as set forth in additional note 3, wherein the preprocessing includes performing an independent component analysis that extracts a small number of independent information sources from each electrode signal after noise removal.

(Additional note 5)

The speech imagery recognition method as set forth in any one of additional notes 1 to 4, further comprising recognizing speech based on the phoneme-feature vector time sequence.

9

(Additional note 6)

The speech imagery recognition method as set forth in any one of additional notes 1 to 5, further comprising outputting the speech recognized.

(Additional note 7)

The speech imagery recognition method as set forth in additional note 6, further comprising displaying a screen that helps a user adjust the optimal position of the electrodes while performing speech imagery.

(Additional note 8)

The speech imagery recognition method as set forth in any one of additional notes 1 to 7, wherein the analysis process includes extracting the spectral time sequence using a linear predictive analysis.

(Additional note 9)

The speech imagery recognition method as set forth in any one of additional notes 1 to 8, wherein the analysis process includes a process of absorbing a frequency fluctuation based on the discrete signals.

(Additional note 10)

The speech imagery recognition method as set forth in any one of additional notes 1 to 9, wherein the analysis process includes extracting a frequency derived from a peak on a frequency axis as a line spectrum component for each time frame.

(Additional note 11)

The speech imagery recognition method as set forth in any one of additional notes 1 to 10, wherein the extraction process includes outputting a phoneme-likelihood vector time sequence, which is a linguistic feature, through a predetermined convolution operator.

(Additional note 12)

The speech imagery recognition method as set forth in any one of additional notes 1 to 11, implemented by either or both of a mobile terminal and a server.

(Additional note 13)

The speech imagery recognition method as set forth in any one of additional notes 1 to 12, further comprising outputting signals from a plurality of electrodes of a wearing fixture, which are placed over Broca's area.

[Industrial Applicability]

With a speech imagery recognition device, a wearing fixture, a method, and a program according to the embodiments of the present invention, line spectral components as a linguistic representation can be directly extracted from EEG signals during speech imagery and converted into phoneme features. Thus, a brain-computer interface (BCI) can be incorporated into the current framework of speech recognition.

[List of Reference Signs]

1    Speech imagery recognition device
2    EEG input unit
3    Preprocessor
4    Analysis processor
5    Linguistic feature extractor

6    Word/sentence recognizer

7    Post-processing/output unit

**Claims**

1.  A speech imagery recognition device configured to recognize speech from electroencephalogram (EEG) signals during speech imagery, the device comprising:

    an analysis processor configured to analyze discrete signals, which are obtained from EEG signals received from a plurality of electrodes, for each of the electrodes and output a spectral time sequence; and
    an extractor configured to output a phoneme-feature vector time sequence based on the spectral time sequence.

2.  The speech imagery recognition device according to claim 1, further comprising an EEG input unit configured to convert the EEG signals received from the electrodes to the discrete signals.

3.  The speech imagery recognition device according to claim 1 or 2, further comprising a preprocessor configured to subtract an average noise amplitude spectrum from a spectrum of a speech imagery signal obtained by converting the discrete signals to a frequency domain to remove noise from the EEG signals.

4.  The speech imagery recognition device according to claim 3, wherein the preprocessor is further configured to perform an independent component analysis that extracts a small number of independent information sources from each electrode signal after noise removal.

5.  The speech imagery recognition device according to any one of claims 1 to 4, further comprising a recognizer configured to recognize speech based on the phoneme-feature vector time sequence.

6.  The speech imagery recognition device according to any one of claims 1 to 5, further comprising an output unit configured to output the speech recognized by the recognizer.

7.  The speech imagery recognition device according to claim 6, wherein the output unit is further configured to display a screen that helps a user adjust the optimal position of the electrodes while performing speech imagery.

8.  The speech imagery recognition device according to any one of claims 1 to 7, wherein the analysis processor is further configured to extract the spectral time sequence using a linear predictive analysis.

9.  The speech imagery recognition device according to any one of claims 1 to 8, wherein the analysis processor is further configured to perform a process of absorbing a frequency fluctuation based on the discrete signals.

10. The speech imagery recognition device according to any one of claims 1 to 9, wherein the analysis processor is further configured to extract a frequency derived from a peak on a frequency axis as a line spectrum component for each time frame.

11. The speech imagery recognition device according to any one of claims 1 to 10, wherein the extractor is further configured to output a phoneme-likelihood vector time sequence, which is a linguistic feature, through a predetermined convolution operator.

12. The speech imagery recognition device according to any one of claims 1 to 11, further comprising a plurality of electrodes configured to be placed over Broca's area.

13. The speech imagery recognition device according claim 12, further comprising a wearing fixture configured to be worn on the head.

14. The speech imagery recognition device according to any one of claims 1 to 12, comprising either or both of a mobile terminal and a server.

15. A wearing fixture for a speech imagery recognition device configured to recognize speech from electroencephalogram (EEG) signals during speech imagery, the wearing fixture comprising:

a plurality of electrodes configured to be placed over Broca's area; and
a processor configured to output signals from the electrodes,
wherein the speech imagery recognition device is configured to:

    analyze discrete signals, which are obtained from EEG signals output from the processor, for each of the electrodes to output a spectral time sequence; and
    extract and output a phoneme-feature vector time sequence based on the spectral time sequence.

16. A speech imagery recognition method for recognizing speech from electroencephalogram (EEG) signals during speech imagery, the method comprising:

    analyzing discrete signals, which are obtained from EEG signals received from a plurality of electrodes, for each of the electrodes to output a spectral time sequence; and
    extracting and outputting a phoneme-feature vector time sequence based on the spectral time sequence.

17. A program that implements a speech imagery recognition process of recognizing speech from electroencephalogram (EEG) signals during speech imagery, the program causing a computer to:

    analyze discrete signals, which are obtained from EEG signals received from a plurality of electrodes, for each of the electrodes to output a spectral time sequence; and
    extract a phoneme-feature vector time sequence based on a spectral component for each of the electrodes.

# FIG. 1

1

EP 3 973 861 A1

EEG INPUT FROM ELECTRODES
ANALOG SIGNAL
ELECTRODE q, TIME t

2

**EEG INPUT UNIT**

DISCRETE SIGNAL
OF ELECTRODE q
$x_1(q, n)$

3

**PREPROCESSOR**

DISCRETE ELECTRICAL SIGNAL
OF ELECTRODE q
$x_2(q, n)$

4

**ANALYSIS PROCESSOR**

SPECTRAL TIME SERIES
OF ELECTRODE q
$A(q, f, n')$

5

**LINGUISTIC FEATURE EXTRACTOR**

PHONEME FEATURE VECTOR
TIME SERIES
$L(k, n')$

6

**WORD/SENTENCE RECOGNIZER**

WORD (SERIES) OUTPUT

7

**POST-PROCESSING/ OUTPUT UNIT**

OUTPUT DISPLAY

# FIG. 2

9 ELECTRODES
IN BROCA'S AREA

EP 3 973 861 A1

# FIG. 3

FREQUENCY [Hz]

TIME FRAME
(4 msec interval)

(A) BEFORE NOISE REMOVAL

FREQUENCY [Hz]

TIME FRAME
(4 msec interval)

(B) AFTER NOISE REMOVAL

# FIG. 4

SIGNAL SOURCE (WHITE NOISE)

$w(n), W(f)$

| IMPULSE RESPONSE s(n) OF NERVOUS SYSTEM |
| TRANSFER (FREQUENCY) FUNCTION S(f) |

EEG (REGULARITY IS PROVIDED BY NERVOUS SYSTEM)

$x(n)=w(n) \, ☆ \, s(n)$
$X(f)=W(f)S(f)=S(f)$

(a) SPEECH IMAGERY EEG SYNTHESIS MODEL

$H(f)$

EEG

$x(n), X(f)$

$\sigma$

$s(n)$
$S(f)$

RESIDUAL (SIGNAL SOURCE)

$w(n), W(f)$
$W(f)=X(F)H(f)$
$H(f) =W(f)/ X(f)= \sigma /X(f)$

(b) SPEECH IMAGERY EEG ANALYSIS MODEL

EP 3 973 861 A1

# FIG. 5

# FIG. 6

SHORT SINE WAVE
(FREQUENCY $f_1$ Hz)

t

SHORT SINE WAVE
(FREQUENCY $f_2$ Hz)

t

LINE SPECTRUM

$f_1$        $f_2$        FREQUENCY [Hz]

COMPOSITE SHORT SINE WAVE SPECTRUM

# FIG. 7

```
                    ┌──────────┐
                    │  START   │
                    └────┬─────┘
                         │
                         │                S1
                         ▼
              ┌─────────────────────┐        ┌──────────────────────────────┐
              │    RECEIVE DATA     │◄────────│ SPECTRAL TIME SERIES OF      │
              └─────────┬───────────┘        │ ELECTRODE q                  │
                        │                     │ (OR AFTER ICA ANALYSIS)      │
                   A(q,f,n')                  └──────────────────────────────┘
                        │                S2
                        ▼
              ┌─────────────────────┐
              │  ABSORB FREQUENCY   │
              │    FLUCTUATION      │
              └─────────┬───────────┘
                        │
                   A(q,f,n')
                        │                S3
                        ▼
              ┌─────────────────────┐
              │   EXTRACT LINE      │
              │    SPECTRUM         │
              └─────────┬───────────┘
                        │                S4
                        ▼
              ┌─────────────────────┐  A(f,n')  ┌──────────────────────────┐
              │ CALCULATE PHONEME   │◄──────────│ PHONEME UNIT             │
              │ LIKELIHOOD BY       │           │ CONVOLUTION              │
              │ CONVOLUTION         │           │ OPERATOR                 │
              └─────────┬───────────┘           └──────────────────────────┘
                        │
                      L(k)              S5
                        ▼
              ┌─────────────────────┐  L(k,n')  ┌──────────────────────────┐
              │ OUTPUT PHONEME      │──────────►│ PHONEME LIKELIHOOD        │
              │ LIKELIHOOD VECTOR   │           │ TIME SERIES              │
              │ TIME SERIES         │           └──────────────────────────┘
              └─────────┬───────────┘
                        │
                        ▼
                    ┌──────────┐
                    │   END    │
                    └──────────┘
```

# FIG. 8

(A) BEFORE FREQUENCY FLUCTUATION ABSORPTION

(B) AFTER FREQUENCY FLUCTUATION ABSORPTION

FIG. 9

EP 3 973 861 A1

# FIG. 10

(A)

(B)

# FIG. 11

START

STORE DATA — S11

9 ELECTRODES WITH KNOWN PHONETIC CONTEXT (OR FEW AFTER ICA) SPECTRAL TIME SERIES (SYLLABLES k: ka, ki, ku, ke, ko)

$A(k,f,n')$

SYLLABLE EIGENVECTOR $\psi^{cv}(m)$

PERFORM PRINCIPAL COMPONENT ANALYSIS BY SYLLABLE (EIGENVECTOR) — S12

UNKNOWN 9-ELECTRODE SPECTRAL TIME SERIES

PHONEME EIGENVECTOR $\phi^k(m')$

PERFORM INTEGRATION INTO PHONEME-SPECIFIC AUTOCORRELATION MATRIX — S13

$X(q,f,n')$

CALCULATE PHONEME LIKELIHOOD BY CONVOLUTION — S15

$X(f,n')$

EXTRACT REPRESENTATIVE SPECTRAL TIME SERIES — S14

OUTPUT PHONEME LIKELIHOOD — S16

END

# FIG. 12

FREQUENCY [Hz]

EIGENVECTOR OF PHONEME /a/

170
120
70

170
120
70

170
120
70

F7    F5    F3

FT7    FC5    FC3

T7    C5    C3

1    5    9    1    5    9    1    5    9

TIME FRAME NUMBER

(A)

FREQUENCY [Hz]

EIGENVECTOR OF PHONEME /s/

170
120
70

170
120
70

170
120
70

F7    F5    F3

FT7    FC5    FC3

T7    C5    C3

1    5    9    1    5    9    1    5    9

TIME FRAME NUMBER

(B)

EP 3 973 861 A1

# FIG. 13

# FIG. 14

EP 3 973 861 A1

FIG. 15

FIG. 16

# FIG. 17

## INTERNATIONAL SEARCH REPORT

| International application No. |
| --- |
| PCT/JP2020/020342 |

### A. CLASSIFICATION OF SUBJECT MATTER
A61B 5/0476(2006.01)i; G06F 3/01(2006.01)i
FI: G06F3/01 515; A61B5/04 322
According to International Patent Classification (IPC) or to both national classification and IPC

### B. FIELDS SEARCHED

Minimum documentation searched (classification system followed by classification symbols)
A61B5/0476; G06F3/01

| Documentation searched other than minimum documentation to the extent that such documents are included in the fields searched | |
| --- | --- |
| Published examined utility model applications of Japan | 1922–1996 |
| Published unexamined utility model applications of Japan | 1971–2020 |
| Registered utility model specifications of Japan | 1996–2020 |
| Published registered utility model applications of Japan | 1994–2020 |

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
| --- | --- | --- |
| Y<br>A | JP 2-232783 A (NIPPON TELEGRAPH AND TELEPHONE CORP.) 14.09.1990 (1990-09-14) page 4, lower right column, lines 8-17, page 5, upper left column, lines 3-15, fig. 1 | 1-10, 12-17<br>11 |
| Y<br>A | US 2012/0022391 A1 (LEUTHARDT, Eric C.) 26.01.2012 (2012-01-26) paragraph [0028] | 1-10, 12-17<br>11 |
| Y<br>A | JP 2009-297059 A (TOYOTA CENTRAL R&D LABS., INC.) 24.12.2009 (2009-12-24) paragraphs [0020], [0022]-[0029], [0081]-[0087], fig. 1 | 1-10, 12-17<br>11 |
| Y | JP 2017-74356 A (HIROSHIMA UNIVERSITY) 20.04.2017 (2017-04-20) paragraphs [0063]-[0064] | 4 |
| Y | JP 3-128041 A (TAMURA, Hajime) 31.05.1991 (1991-05-31) page 4, lower right column, lines 8-9 | 8 |

☐ Further documents are listed in the continuation of Box C.      ☒ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| 20 July 2020 (20.07.2020) | 04 August 2020 (04.08.2020) |

| Name and mailing address of the ISA/<br>    Japan Patent Office<br>    3-4-3, Kasumigaseki, Chiyoda-ku,<br>    Tokyo 100-8915, Japan | Authorized officer<br><br>Telephone No. |
| --- | --- |

Form PCT/ISA/210 (second sheet) (January 2015)

**INTERNATIONAL SEARCH REPORT**
Information on patent family members

International application No.

PCT/JP2020/020342

| Patent Documents referred in the Report | Publication Date | Patent Family | Publication Date |
|---|---|---|---|
| JP 2-232783 A | 14 Sep. 1990 | (Family: none) | |
| US 2012/0022391 A1 | 26 Jan. 2012 | WO 2012/012746 A2 | |
| JP 2009-297059 A | 24 Dec. 2009 | (Family: none) | |
| JP 2017-74356 A | 20 Apr. 2017 | US 2018/0303370 A1 paragraphs [0097]-[0098] WO 2017/064826 A1 EP 3360480 A1 | |
| JP 3-128041 A | 31 May 1991 | (Family: none) | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2008204135 A

**Non-patent literature cited in the description**

- **HEGER D. et al.** Continuous Speech Recognition from ECoG. *Interspeech2015,* 2015, 1131-1135
- **INDEFREY, P et al.** The spatial and temporal signatures of word production components. *Cognition,* 2004, vol. 92, 101-144
- **BOUCHARD K.E. et al.** Functional organization of human sensorimotor cortex for speech articulation. *Nature,* 2013, vol. 495, 327-332
- **GIROLAMI M.** Advances in Independent Component Analysis. Springer, 2000
- **DURBIN, J.** The fitting of time series models. *Rev. Inst. Int. Stat.,* 1960, vol. 28, 233-243